(19) Europäisches Patentamt
European Patent Office
Office européen des brevets

(11) Veröffentlichungsnummer: **0 047 888**
**B1**

(12) # EUROPÄISCHE PATENTSCHRIFT

(45) Veröffentlichungstag der Patentschrift: **29.06.83**

(51) Int. Cl.³: **C 07 C 121/75**, C 07 C 121/66, C 07 C 85/08, C 07 C 120/00

(21) Anmeldenummer: **81106607.5**

(22) Anmeldetag: **26.08.81**

(54) **Verfahren zur Herstellung basisch substituierter Phenylacetonitrile.**

(30) Priorität: **11.09.80 DE 3034221**

(43) Veröffentlichungstag der Anmeldung: **24.03.82 Patentblatt 82/12**

(45) Bekanntmachung des Hinweises auf die Patenterteilung: **29.06.83 Patentblatt 83/26**

(84) Benannte Vertragsstaaten: **AT BE CH DE FR GB IT LI LU NL SE**

(56) Entgegenhaltungen:
**AT-B-331 217**
**DE-A-2 059 923**
**DE-A-2 263 527**
**DE-B-2 631 222**
**DE-C-894 118**

**"Organic Reactions", 2. Auflage, Band V 1952, JOHN WILEY & SONS, New York**

**SYNTHESIS, Nr. 9, September 1974, Stuttgart, G.D. CHERAYIL "Dimethylation of Phenethylamines", Abstract Nr. 3975, Seiten 676 bis 678, und J. Pharm. Sci, Band 62, 1973, Seite 2054**

(73) Patentinhaber: **BASF Aktiengesellschaft, Carl-Bosch-Strasse 38, D-6700 Ludwigshafen (DE)**

(72) Erfinder: **Kastner, Gerhard, Dr., Kalmusstrasse 12, D-6700 Ludwigshafen-Maudach (DE)**
Erfinder: **Siegel, Hardo, Dr., Hans-Purrmann-Allee 25, D-6720 Speyer (DE)**
Erfinder: **Geiss, Karl-Heinz, Dr., Kirchenstrasse 8, D-6711 Beindersheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**EP 0 047 888 B1**

# Verfahren zur Herstellung basisch substituierter Phenylacetonitrile

Die Erfindung betrifft ein neues Verfahren zur Herstellung basisch substituierter Phenylacetonitrile.

Es ist bekannt, dass basisch substituierte Phenylacetonitrile coronar-vasodilatatorische und antiarrhythmische Eigenschaften besitzen und daher wertvolle Arzneimittel zur Behandlung verschiedener Koronarkrankheiten sind (DE-PS 1154810, Verapamil).

Für die Herstellung basisch substituierter Phenylacetonitrile sind bereits verschiedene Verfahren bekannt (DE-PS 1154810, DE-PS 1158083, DE-OS 2059923, DE-AS 2263527, DE-AS 2631222, DD-PS 119579).

In der DE-AS 2631222 ist ein Verfahren zur Herstellung basisch substituierter Phenylacetonitrile beschrieben, welches darin besteht, dass man zunächst ein Isopropylbenzylcyanid mit einem ω-Halogenacetal kondensiert, dann das so erhaltene Nitrilaldehydacetal mit wässriger Säure in den Nitrilaldehyd überführt und diesen mit einem Amin einer hydrierenden Kondensation unterwirft.

Es wurde nun gefunden, dass man den Nitrilaldehyd auch nach Leuckart-Wallach mit dem Amin kondensieren kann.

Gegenstand der Erfindung ist ein Verfahren zur Herstellung basisch substituierter Phenylacetonitrile der Formel I

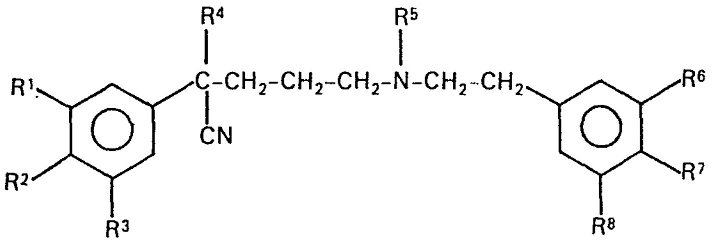

I,

worin
$R^1$, $R^2$ und $R^3$ Wasserstoff- oder Halogenatome, Trifluormethyl- oder $C_{1-6}$-Alkoxygruppen, wobei $R^1$ und $R^2$ zusammen auch eine Methylendioxogruppe sein können,
$R^4$ und $R^5$ $C_{1-6}$-Alkylgruppen,
$R^6$ und $R^7$ $C_{1-6}$-Alkoxygruppen oder zusammen eine Methylendioxogruppe und
$R^8$ ein Wasserstoffatom oder eine $C_{1-6}$-Alkoxygruppe bedeuten,
welches darin besteht, dass man einen Aldehyd der Formel II

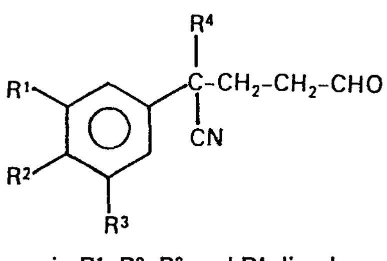

II,

worin $R^1$, $R^2$, $R^3$ und $R^4$ die oben angegebene Bedeutung besitzen, mit 0,8–1,5 Äquivalenten eines Amins der Formel III

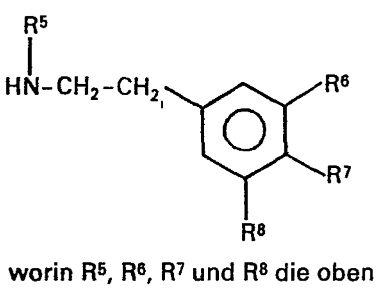

III,

worin $R^5$, $R^6$, $R^7$ und $R^8$ die oben angegebene Bedeutung besitzen, in Gegenwart von 0,8–1,5 Äquivalenten Ameisensäure bei 20 bis 150°C umsetzt.

Die Umsetzungen der Verbindungen II und III können ohne oder in Gegenwart eines inerten Lösungsmittels durchgeführt werden. Geeignete Lösungsmittel sind beispielsweise Wasser, niedere aliphatische Alkohole und Diole – wie Methanol, Ethanol, n-Propanol, Isopropanol, n-Butanol, Isobutanol, Ethylenglykol, 1.2-Propylenglykol, 1.3-Propylenglykol –, niedrige gesättigte Dialkylether, Monoalkylglykolether, Dialkylglykolether oder gesättigte cyclische Ether – wie Diethylether, Methyltertiärbutylether, Dipropylether, Ethylenglykolmonomethylether, Ethylenglykolmonoethylether, Diethylenglykoldimethylether, Tetrahydrofuran, Dioxan –, niederaliphatische Ester niederaliphatischer Carbonsäuren – wie z.B. Essigsäuremethylester, Essigsäureethylester, Essigsäureisobutylester, Propionsäureethylester –, Amide niederaliphatischer Carbonsäuren – wie z.B. Acetamid, N-Methylacetamid, N,N-Dimethylacetamid, N,N-Dimethylformamid –, Lactame – wie Pyrrolidon, N-Methylpyrrolidon, Caprolactam –, Halogenkohlenwasserstoffe, insbesondere Chlorkohlenwasserstoffe – wie z.B. Methylenchlorid, Chloroform, 1.2-Dichlorethan, 1,1,2,2- oder 1,1,1,2-Tetrachlorethan, 1,1,1-Trichlorethan, Tetrachlorethylen, Propylchlorid, Amylchlorid, Chlorbenzol, o-, p- und m-Dichlorbenzol,o-, m-,p-Chlortoluol,1,2,4-Trichlorbenzol –; aromatische Kohlenwasserstoffe – wie Benzol –, alkylierte aromatische Kohlenwasserstoffe – wie Toluol, Xylol – oder aliphatische Kohlenwasserstoffe – wie Hexan, Heptan, Octan, Dodecan, Cyclohexan. Die Umsetzungen können auch in Mischungen der genannten Lösungsmittel durchgeführt werden.

Bevorzugt sind von diesen Lösungsmitteln Wasser, Alkohole mit 1–6 Kohlenstoffatomen – wie Methanol, sek.-Butanol –, niedrige gesättigte Dialkylether und cyclische Ether – wie Diethylether, Tetrahydrofuran, Dioxan –, niederaliphati-

sche Ester niederaliphatischer Carbonsäuren mit bis zu 8 Kohlenstoffatomen – wie Essigsäureethylester, Essigsäureisobutylester –, N,N-Dialkylamide niederaliphatischer Carbonsäuren – wie Dimethylformamid, Dimethylacetamid –, Chlorkohlenwasserstoffe – wie Methylenchlorid, Chloroform, insbesondere Chlorbenzol, o-, m-, p-Chlortoluol –, aromatische, gegebenenfalls alkylsubstituierte Kohlenwasserstoffe – wie insbesondere Benzol, Toluol und Xylol – und aliphatische Kohlenwasserstoffe – wie Hexan, Heptan, Octan, Cyclohexan.

In der bevorzugten Form der Ausführung werden die Aldehyde II mit 0,9–1,3, vorzugsweise 1,0–1,1 Äquivalenten Amin III und 0,9–1,3, vorzugsweise 1,0–1,2 Äquivalenten Ameisensäure bei Temperaturen zwischen 50 und 120 °C, vorzugsweise 50°C, bis zur Rückflusstemperatur des verwendeten Lösungsmittels umgesetzt.

Die Aufarbeitung der Reaktionsgemische erfolgt nach einer der üblichen Methoden, wie Extraktion, Kristallisation, Destillation und/oder Chromatographie.

Das Verfahren liefert hohe Ausbeuten und führt zu Produkten hoher Reinheit. Gegenüber der katalytischen Hydrierung hat die erfindungsgemässe Leuckart-Wallach-Reaktion den Vorteil, dass keine technisch aufwendigen Druckgefässe benötigt werden und dass die Reaktionsführung wesentlich einfacher ist.

Es war nicht zu erwarten, dass die Leuckart-Wallach-Reaktion der Aldehyde II die Produkte I ergibt. Es ist nämlich bekannt, dass Homoveratrylamin IV und seine N-monoalkylierten Derivate unter den Bedingungen der Leuckart-Wallach-Reaktion nicht die gewünschten alkylierten Amine V liefern, sondern Tetrahydroisochinoline VI bilden:

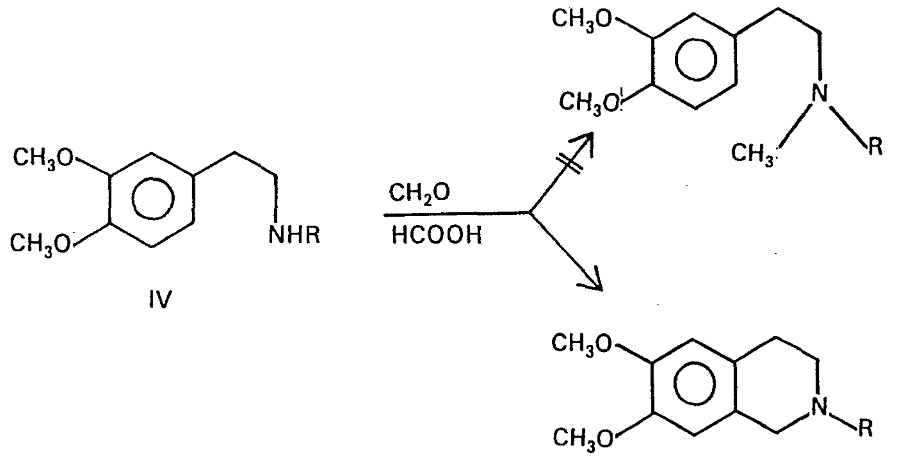


IV

V

VI

Diese Ringschlussrekation ist als Pictet-Spengler-Reaktion bekannt [Org. Reactions 6, S. 151–190 (1951)]. Sie verläuft besonders gut bei elektronenreichen Aromaten.

In Houben-Weyl XI/1, S. 644 ist der unterschiedliche Verlauf der reduktiven Alkylierung von N-3,4-Dimethoxyphenylethyl-N-3,4-dimethoxybenzylamin VII bei katalytischer Reduktion und bei Reduktion unter Leuckart-Wallach-Bedingungen eindrucksvoll demonstriert. Während bei der Umsetzung von VII mit Formaldehyd/Raney-Ni/Wasserstoff das gewünschte methylierte Amin VIII entsteht, wurde unter Leuckart-Wallach-Bedingungen als einziges Produkt das Tetrahydroisochinolinderivat IX erhalten:

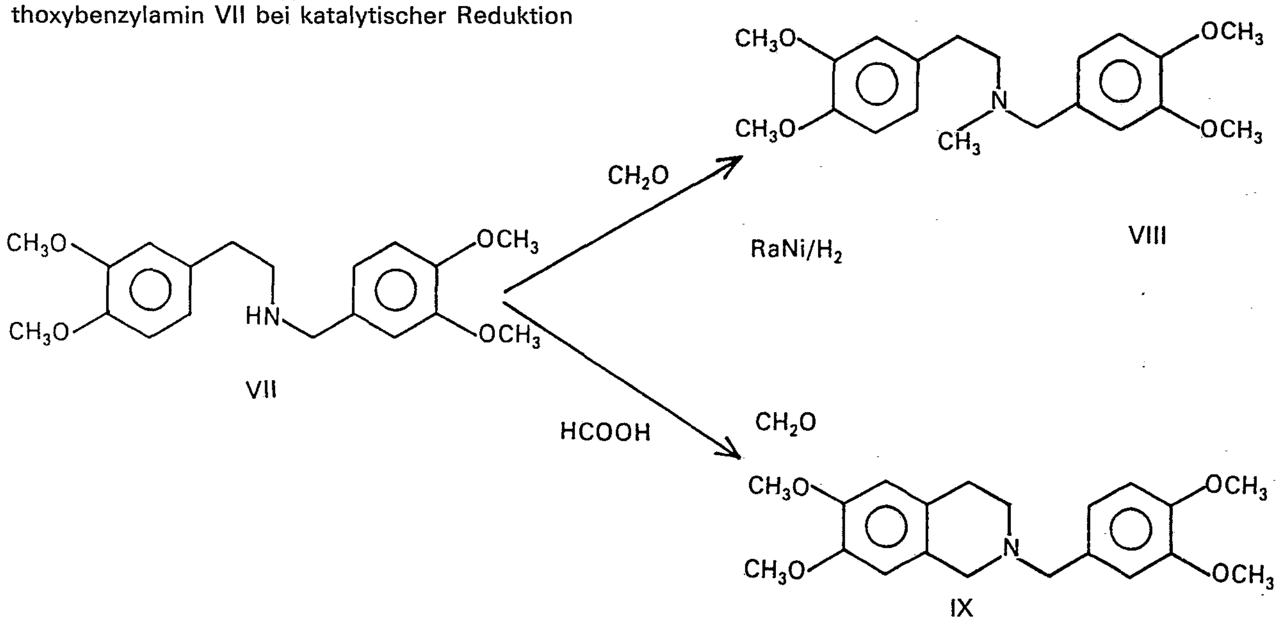


VII

VIII

IX

Homoveratrylamin und 3,4,5-Trimethoxyphenylethylamin verhalten sich unter Leuckart-Wallach-Bedingungen analog VII (Houben-Weyl XI/1, S. 652). R. Baltzly [J. Amer. Chem. soc. 75 (1953), 6038-6039] versuchte, die Methylierung von Homoveratrylamin bei sehr niedrigen Säurekonzentrationen durchzuführen, indem er zu einer am Rückfluss kochenden Lösung von Homoveratrylamin und Formaldehyd Ameisensäure so zudosierte, dass der pH-Wert nahe bei 7 blieb und erst gegen Ende der Reaktion auf pH 5 fiel. Trotz dieser Vorkehrungen betrug die Ausbeute an N,N-Dimethylhomoveratrylamin nur 44 %. Ausserdem wurden 14 % 2-Methyl-6,7-dimethoxytetrahydroisochinolin erhalten. Der Autor schliesst aus seinen Versuchen, dass bei Vorliegen der für die Cyclisierung günstigen Struktur eines Phenylethylamins die Reaktion nicht so gelenkt werden kann, dass die Cyclisierung vollkommen zurückgedrängt wird.

Weiter ist es bekannt, dass Phenylethylamine mit elektronenreichen Aromaten (z.B. Homoveratrylamin) in Gegenwart von Säure nicht nur mit Formaldehyd, sondern auch mit aliphatischen, aromatischen und araliphatischen Aldehyden in guten bis sehr guten Ausbeuten entsprechend substituierte Tetrahydroisochinoline bilden [Organic Reactions 6, 151-190 (1951)]. Es war daher zu erwarten, dass bei der Umsetzung von Aldehyden der allgemeinen Formel II mit Aminen der allgemeinen Formel III unter Leuckart-Wallach-Bedingungen im wesentlichen Tetrahydroisochinoline gebildet werden.

Beispiel 1

Eine Lösung von 275 g α-Isopropyl-α-(γ-oxopropyl)-veratrylcyanid und 194 g N-Methylhomoveratrylamin (MeHVA) in 1,6 l Toluol wird mit 51 g 90%iger Ameisensäure versetzt und 1 Stunde unter Rückfluss gekocht.

Nach Abkühlen der Reaktionslösung wird die wässrige Phase mit Schwefelsäure auf pH 2-4 eingestellt und mit Toluol extrahiert. Anschliessend wird mit NaOH neutralisiert und mit Toluol extrahiert. Die Toluolphase wird mit Wasser gewaschen und im Vakuum eingedampft. Der Rückstand wird in Isopropanol in das Hydrochlorid übergeführt. Man erhält nach Umkristallisieren aus Isopropanol 395 g (88 %) α-Isopropyl-α-[(N-methyl-N-homoveratryl-)-γ-aminopropyl]-3,4-dimethoxyphenylacetonitrilhydrochlorid, Fp. 141 bis 143 °C.

Beispiel 2 bis 12

Analog Beispiel 1 wurden die Beispiele 2-12 gemäss Tabelle 1 durchgeführt.

Tabelle 1

| Beispiel Nr. | Äquivalente MeHVA | Äquivalente HCOOH |
|---|---|---|
| 2 | 0,9 | 0,9 |
| 3 | 1,0 | 1,0 |
| 4 | 1,0 | 1,1 |

Tabelle 1 (Fortsetzung)

| Beispiel Nr. | Äquivalente MeHVA | Äquivalente HCOOH |
|---|---|---|
| 5 | 1,0 | 1,2 |
| 6 | 1,0 | 1,3 |
| 7 | 1,0 | 1,5 |
| 8 | 1,05 | 1,05 |
| 9 | 1,1 | 1,1 |
| 10 | 1,1 | 1,3 |
| 11 | 1,5 | 1,0 |
| 12 | 1,5 | 1,5 |

Man erhielt in 72 bis 86%iger Ausbeute dasselbe Produkt wie in Beispiel 1. Beispiel 8 lieferte das Produkt mit bester Reinheit und bester Ausbeute. Die Beispiele 3-5 und 11 lieferten das Produkt ebenfalls in bester Reinheit.

Beispiele 13-17

Analog Beispiel 1 wurden die Beispiele 13-17 gemäss Tabelle 2 bei unterschiedlicher Reaktionstemperatur durchgeführt.

Tabelle 2

| Beispiel Nr. | Temperatur (°C) | Reaktionszeit |
|---|---|---|
| 13 | 20 | ≧10 Std. |
| 14 | 50 | 5 Std. |
| 15 | 70 | 2-4 Std. |
| 16 | 80 | 1 Std. |
| 17 | 100 | 1 Std. |

Man erhielt in 70 bis 86%iger Ausbeute dasselbe Produkt wie in Beispiel 1.

Beispiele 18-25

Analog Beispiel 1 wurden die Beispiele 18-25 gemäss Tabelle 3 durchgeführt, Toluol wurde jedoch durch andere Lösungsmittel ersetzt.

Tabelle 3

| Beispiel Nr. | Lösungsmittel |
|---|---|
| 18 | Methanol |
| 19 | sek.-Butanol |
| 20 | sek.-Butanol/$H_2$ 1:4 |
| 21 | Tetrahydrofuran |
| 22 | Dioxan |
| 23 | Essigsäureethylester |
| 24 | Dimethylformamid/$H_2O$ 1:4 |
| 25 | Chloroform |

Man erhielt Ausbeuten von 70 bis 85 %.

Beispiel 26

Analog Beispiel 1 wurde aus 305 g α-Isopropyl-α-(γ-oxopropyl)-3,4,5-trimethoxybenzylnitril und 194 g N-Methylhomoveratrylamin in 51 g 90%iger Ameisensäure 448 g (86%) α-Isopropyl-α-[(N-methyl-N-homoveratryl)-γ-aminopropyl]-3,4,5-trimethoxyphenylacetonitrilhydrochlorid vom Fp. 145 bis 147 °C erhalten.

Beispiel 27

Analog Beispiel 1 wurden aus 313 g α-Isopropyl-α-(γ-oxopropyl)-3-trifluoromethylbenzylnitril und 194 g N-Methylhomoveratrylamin in 46 g Ameisensäure 470 g (84%) α-Isopropyl-α-[(N-Methyl-N-homoveratryl)-γ-aminopropyl]-3-trifluoromethylphenylacetonitrilamidosulfonat vom Fp. 115 bis 117 °C erhalten.

## Patentanspruch

Verfahren zur Herstellung basisch substituierter Phenylacetonitrile der Formel I

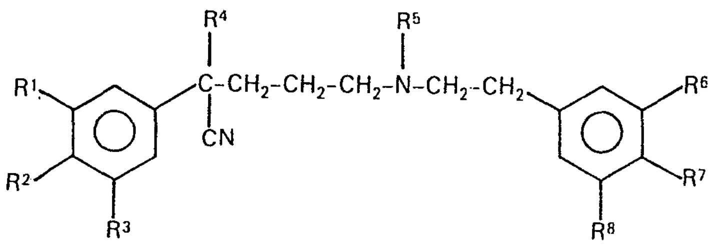

I,

worin
$R^1$, $R^2$ und $R^3$ Wasserstoff- oder Halogenatome, Trifluormethyl- oder $C_{1-6}$-Alkoxygruppen, wobei $R^1$ und $R^2$ zusammen auch eine Methylendioxogruppe sein können,
$R^4$ und $R^5$ $C_{1-6}$-Alkylgruppen,
$R^6$ und $R^7$ $C_{1-6}$-Alkoxygruppen oder zusammen eine Methylendioxogruppe und
$R^8$ ein Wasserstoffatom oder eine $C_{1-6}$-Alkoxygruppe bedeuten,
dadurch gekennzeichnet, dass man einen Aldehyd der Formel II

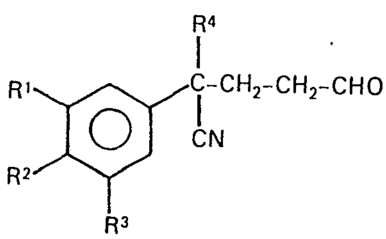

II,

worin $R^1$, $R^2$, $R^3$ und $R^4$ die oben angegebene Bedeutung besitzen, mit 0,8–1,5 Äquivalenten eines Amins der Formel III

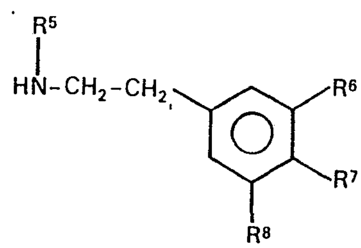

III,

worin $R^5$, $R^6$, $R^7$ und $R^8$ die oben angegebene Bedeutung besitzen, in Gegenwart von 0,8–1,5 Äquivalenten Ameisensäure bei 20 bis 150 °C umsetzt.

## Claim

A process for the preparation of phenylacetonitriles, carrying basic substituents, of the formula I

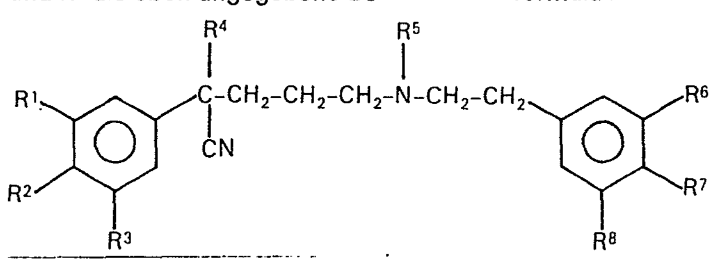

I,

where
$R^1$, $R^2$ and $R^3$ are hydrogen, halogen, trifluoromethyl or $C_1$–$C_6$-alkoxy, and $R^1$ and $R^2$ together can also be methylenedioxy, $R^4$ and $R^5$ are $C_1$–$C_6$-alkyl, $R^6$ and $R^7$ are $C_1$–$C_6$-alkoxy or together are methylenedioxy, and $R^8$ is hydrogen or $C_1$–$C_6$-alkoxy, wherein an aldehyde of the formula II

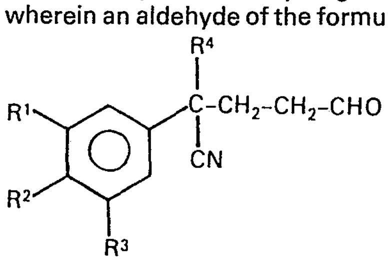

II,

where $R^1$, $R^2$, $R^3$ and $R^4$ have the above meanings, is reacted with from 0.8 to 1.5 equivalents of an amine of the formula III

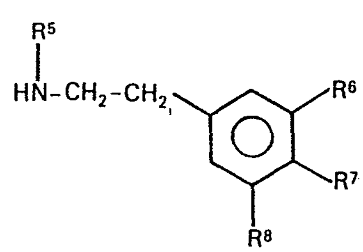

III,

where $R^5$, $R^6$, $R^7$ and $R^8$ have the above meanings, in the presence of from 0.8 to 1.5 equivalents of formic acid at from 20 to 150 °C.

## Revendication

Procédé de préparation de phényl-acétonitriles à substitution basique de la formule I

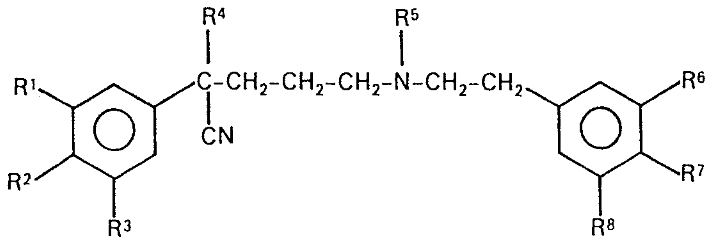

I,

dans laquelle
$R^1$, $R^2$ et $R^3$ représentent des atomes d'hydrogène ou d'halogène ou des groupes trifluoro-méthyle ou alcoxy en $C_1$ à $C_6$, $R^1$ et $R^2$ pouvant aussi former ensemble un groupe méthylène-dioxo;
$R^4$ et $R^5$ désignent des radicaux alcoyle en $C_1$ à $C_6$;
$R^6$ et $R^7$ désignent des groupes alcoxy en $C_1$ à $C_6$ ou constituent ensemble un groupe méthylène-dioxo et
$R^8$ représente un atome d'hydrogène ou un groupe alcoxy en $C_1$ à $C_6$,
caractérisé en ce que l'on fait réagir un aldéhyde de la formule II

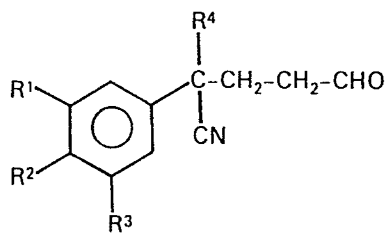

II,

dans laquelle $R^1$, $R^2$, $R^3$ et $R^4$ possèdent la signification définie ci-dessus, avec 0,8 à 1,5 équivalent d'une amine de la formule III

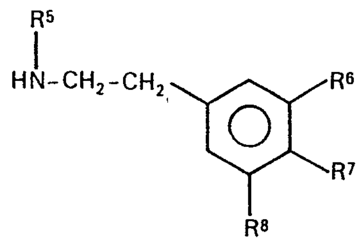

III,

dans laquelle $R^5$, $R^6$, $R^7$ et $R^8$ possèdent la signification définie ci-dessus, entre 20 et 150 °C en présence de 0,8 à 1,5 équivalent d'acide formique.